# EUROPÄISCHE PATENTSCHRIFT

(11) **EP 1 420 701 B1**
(45) Veröffentlichungstag und Bekanntmachung des Hinweises auf die Patenterteilung: **14.01.2015**
(21) Anmeldenummer: 02767376.3
(22) Anmeldetag: 13.08.2002
(51) Int. Cl.: A61B 17/12

(54) **VORRICHTUNG ZUR IMPLANTATION VON OCCLUSIONSMITTELN**
DEVICE FOR THE IMPLANTATION OF OCCLUSION MEANS
DISPOSITIF POUR IMPLANTER DES ELEMENTS D'OCCLUSION

(30) Priorität: 27.08.2001 DE 20113741 U; 24.07.2002 DE 10233728
(43) Veröffentlichungstag der Anmeldung: 26.05.2004
(73) Patentinhaber: Dendron GmbH, 44799 Bochum (DE)
(72) Erfinder: MONSTADT, Hermann, 44797 Bochum (DE); HENKES, Hans, 44797 Bochum (DE); DENK, Marion, 44803 Bochum (DE)
(74) Vertreter: Behrendt, Arne
(86) Internationale Anmeldenummer: PCT/EP2002/009063
(87) Internationale Veröffentlichungsnummer: WO 2003/017852

(56) Entgegenhaltungen:
- WO-A-00/74577
- WO-A-01/32085
- US-A- 5 895 385
- US-A- 5 941 888

## Beschreibung

Die Erfindung betrifft eine Vorrichtung zur Implantation von elektrolytisch ablösbaren Occlusionsmitteln in Körperhohlräumen oder Blutgefäßen.

Der Einsatz endovaskulärer Techniken zur Occlusion von Körperhohlräumen oder Gefäßen wie Arterien, Venen, Eileitern oder vaskulären Fehlbildungen wie z. B. vaskulärer Aneurysmen ist bekannter Stand der Technik. Das Occlusionsmittel wird dabei in der Regel mit Hilfe einer Einführhilfe durch einen Katheter in den zu occludierenden Hohlraum eingeführt und deponiert.

Die zur Deponierung notwendige Abtrennung des Occlusionsmittels gestaltet sich dabei technisch besonders problematisch, denn die hierzu notwendige Vorrichtung muß einerseits möglichst klein gehalten sein, um durch den geringen Durchmesser des Katheters an die Zielorte geführt werden zu können, andererseits muß sie eine zuverlässige Abtrennung des Occlusionsmittels bewirken, da es sonst bei Rückführung des Katheters zu einer ungewollten Entfernung des Occlusionsmittels aus dem zu occludierenden Hohlraum und somit zu Verletzung und/oder Ruptur der Hohlraum- oder Gefäßwandung kommen kann.

Mechanische Verfahren zur Abtrennung des Occlusionsmittels von der Einführhilfe sind zwar mit einem geringen Zeitaufwand verbunden, die technisch bedingte Starrheit der Verbindung von Occlusionsmittel und Einführhilfe erschwert jedoch die Implantateinbringung. Zudem birgt die durch die Starrheit bedingte geringe Belastbarkeit der Verbindung ein nicht unerhebliches Risiko der vorzeitigen Trennung von Einführhilfe und zu implantierendem Occlusionsmittel. Weiterhin muß bei der mechanischen Trennung von Einführdraht und Occlusionsmittel Energie übertragen werden (in der Regel durch Rotation des Einführdrahtes), durch die auch das zu implantierende Occlusionsmittel aus der gewünschten Position verlagert werden kann.

Die elektrolytische Ablösung von Edelstahl-Drahtspitzen im Blut während der Transkatheter Elektrokoagulation von Blutgefäßen oder Fehlbildungen derselben, wurde zum ersten mal 1979 durch Thompson et al und McAlister et al beschrieben (Radiology 133:335-340, November 1979, AJR 132:998-1000, Juni 1979).

Darauf aufbauend beschreibt die EP 0 484 468 eine Vorrichtung zur Implantation eines Occlusionsmittels, basierend auf der elektrolytisch korrodierbaren Ausbildung der Drahtspitze des Führungsdrahtes an der Verbindung zwischen Führungsdraht und Occlusionsmittel. Diese Vorrichtung nutzt zwar auf elegante Weise die zur Elektrothromboisierung an die als Anode dienende Occlusions-mittel angelegte elektrische Spannung für die gleichzeitige Abtrennung der Drahtspitze und der daran befindlichen Occlusionsmittel. Aufgrund der zur sicheren Führung des Implantates notwendigen vergleichsweise massiven Ausbildung des Führungsdrahtes kann jedoch auch die zur Ablösung bestimmte distale Führungsdrahtspitze nur schlecht an die zur schnellen und somit sicheren Ablösung notwendigen Erfordernisse angepaßt werden:

So können zur Ausbildung des Führungsdrahtes beispielsweise nur Materialien eingesetzt werden, welche eine besonders hohe Stabilität aufweisen, damit die sichere Führung des einzuführenden Occlusionsdrahtes durch den Führungsdraht möglich ist. Die Werkstoffauswahl für die Ausbildung der elektrolytischen Ablösestelle ist daher sehr eingeschränkt.

Auch kann die Dimensionierung der Ablösestelle nur eingeschränkt an für eine schnelle Ablösung geeignete Maße angepaßt werden: Bei den im Stand der Technik vorkommenden Vorrichtungen zur elektrolytischen Ablösung von Occlusionsmitteln sind Occlusionsmittel und Führungsdraht nicht aus einem Stück gefertigt, sondern in der Regel mechanisch miteinander verbunden. Diese Ausbildung birgt den Nachteil, daß zur Gewährleistung ausreichender Stabilität im proximalen Bereich des Führungsdrahtes und zwecks Ermöglichung der elektrolytisch korrosiven Auflösung der Drahtspitze im distalen Bereich des Drahtes der Führungsdraht mittels aufwendiger Schleifverfahren zur Spitze hin verjüngt werden muß. Dieser korrodierbare Bereich der Drahtspitze des Führungsdrahtes an der Verbindungsstelle zwischen Führungsdraht und Occlusionsmittel kann jedoch zur Gewährleistung ausreichender Stabilität der Verbindungsstelle einen gewissen Mindestdurchmesser von etwa 0,05 mm nicht unterschreiten, da er einer hohen Biegebeanspruchung unterliegt. Die die Verbindungsstelle zwischen Occlusionsmittel und Führungsdraht darstellende korrodierbare Drahtspitze ist daher recht starr und benötigt zur elektrolytisch korrosiven Auflösung relativ lange Zeiten.

Da der Stand der Technik bisher keine hinsichtlich Kostenaufwand und Sicherheit befriedigende Möglichkeit zur endovaskulären Deponierung von Occlusionsmitteln bietet, liegt der Erfindung die Aufgabe zugrunde, eine Vorrichtung bereitzustellen, die es auf möglichst schnellem, effektivem und sicherem Wege erlaubt, Occlusionsmittel in Körperhöhlen oder Gefäßen zu deponieren.

Diese Aufgabe wird erfindungsgemäß durch eine Vorrichtung gemäß Anspruch 1 gelöst.

Die Erfindung ist in Anspruch 1 definiert. Eine Vorrichtung gemäß dem Oberbegriff des Anspruchs 1 ist aus der WO-A-01/32085 bekannt.

Da die elektrolytisch korrodierbare Stelle der erfindungsgemäßen Vorrichtung einen Teil des Occlusionsmittels selbst darstellt, unterliegt sie im Vergleich zu den herkömmlichen, starren elektrolytisch ausgebildeten Verbindungen zwischen Führungsdraht und Occlusionsmittel einer wesentlichen geringeren Biegebeanspruchung während des Implantationsprozesses. Diese geringe Biegebeanspruchung ermöglicht den Einsatz von elektrolytisch korrodierbaren Stellen mit wesentlich geringeren Durchmessern als im Stand der Technik, was zu einer verbesserten und schnelleren elektrolytischen Ablösbarkeit des Occlusionsmittels führt. Solche geringen Durchmesser von weniger als 0,05 mm sind durch dafür geeignete, z. B. mechanische Verfahren zu erreichen.

Ein weitere Vorteil der Ausbildung der elektrolytisch korrodierbaren Stelle der erfindungsgemäßen Vorrichtung in dem Occlusionsmittel selbst gegenüber der im Stand der Technik vorkommenden Ablösung der Führungsdrahtspitze ist die wesentlich größere Auswahl an Materialien, welche zur Ausbildung der korrodierbaren Ablösestellen eingesetzt werden können. Im Gegensatz zur herkömmlichen Ablösung der Führungsdrahtspitze, muß die in dem Occlusionsmittel angesiedelte elektrolytisch korrodierbare Stelle der erfindungsgemäßen Vorrichtung keine besonders große Stabilität aufweisen, es können daher auch weniger stabile, flexiblere Werkstoffe eingesetzt werden, sofern sie korrodierbar und körperverträglich sind.

Proximal ist an dem Occlusionsmittel eine Einführhilfe in Form eines Führungsdrahtes angeordnet. Der separate Führungsdraht hat den Vorteil, daß er aus gegenüber dem Occlusionsmittel kostengünstigeren Materialien ausgebildet werden kann, zumal er nicht in Kontakt mit dem Körpergewebe kommt. Die Ausgestaltung des Führungsdrahtes wird dabei vorzugsweise so vorgenommen, daß sie eine gute Steuerung des Occlusionsmittels durch den Katheter erlaubt, was zur besseren Deponierbarkeit beiträgt.

Bei einer solchen Ausgestaltung der erfindungsgemäßen Vorrichtung sind Einführhilfe und Occlusionsmittel vorzugsweise durch Löt- und/oder Klebe-und/oder Schweißvorgänge und/oder mechanische Verbindungen miteinander verbunden. Es handelt sich um im Stand der Technik bekannte Verbindungsverfahren, die sich durch Einfachheit und die Stabilität der so herbeigeführten Verbindung auszeichnen.

In einer weiteren zweckmäßigen Ausführungsform können Führungsdraht und Occlusionsmittel der erfindungsgemäßen Vorrichtung als Teile des selben Drahtes ausgebildet sein. Diese Ausführungsform zeichnet sich durch besondere Stabilität aus und kann sehr kostengünstig sein, da vorgenannte Anbindungsverfahren des Führungsdrahtes mit dem Occlusionsmittel wegfallen.

Das Occlusionsmittel weist dabei grundsätzlich wenigstens eine elektrolytisch korrodierbare Stelle in Form eines Ablöseelements oder Formteils auf.

Die Ausführungsform mit wenigstens einer elektrolytisch korrodierbaren Stelle im Occlusionsmittel weist den Vorteil auf, in der Herstellung besonders kostengünstig zu sein und dennoch gegenüber den vorgenannten Vorrichtungen des Standes der Technik die vorstehend erwähnten Vorteile aufzuweisen. Die elektrolytisch korrodierbare Stelle kann dabei grundsätzlich an jeder technisch zweckmäßigen Position des Occlusionsmittels angeordnet sein. Die optimale Positionierung kann dabei vom zuständigen Durchschnittsfachmann einfach bestimmt werden.

Ausführungsformen mit zwei oder mehreren elektrolytisch korrodierbaren Stellen im Occlusionsmittel weisen darüber hinaus den Vorteil auf, daß hier variabel dimensionierbare Längen des Occlusionsmittels zur Implantierung im Körper abgetrennt und deponiert werden können.

Die Integration einer Mehrzahl von elektrolytisch korrodierbaren Stellen in das Occlusionsmittel bietet gegenüber herkömmlichen Vorrichtungen zur elektrolytischen Ablösung von Occlusionsmitteln überdies den Vorteil, daß während eines Implantationsvorganges nicht nur eine sondern mehrere Längen derselben Mittel nacheinander abgelöst und in den zu occludierenden Hohlraum eingebracht werden können. Das spart nicht nur Kosten und Zeit, sondern dient außerdem der weiteren Minimierung des Operationsrisikos.

Die eingangs erwähnten herkömmlichen Vorrichtungen zur Implantation von Occlusionsimplantaten weisen allesamt den Nachteil auf, daß nur Implantate vordefinierter Länge abgetrennt werden können. Es muß also in der Regel vor der unmittelbaren Implantateinbringung anhand der Größe des zu occludierenden Hohlraumes die Länge, d. h. longitudinale Ausdehnung der einzusetzenden Occlusionsmittel vordefiniert werden. Da die unregelmäßige Ausbildung der zu occludierenden Körperhohlräume jedoch die Einschätzung der zur Füllung notwendigen Länge der einzusetzenden Occlusionsmittel erschwert, kann es dazu kommen, daß zu lange oder zu kurze Occlusionsmitteln in den zu occludierenden Hohlraum eingeführt werden, was eine unvollständige Occludierung einerseits bzw. der Beschädigung oder Ruptur der Wandung des zu occludierenden Hohlraumes oder angrenzender Gefäße andererseits bedingen kann.

Diese Ausführungsform der Erfindung mit mehreren elektrolytisch korrodierbaren Stellen beruht auf Experimenten der Erfinder, die ergaben, daß bei Anlegen eines Stromes an eine erfindungsgemäße Vorrichtung sich überraschenderweise spezifisch die dem distalen Ende des Katheters am nächsten liegende elektrolytisch korrodierbar ausgebildete Stelle des Occlusionsmittels durch Elektrolyse löst. Diese Spezifität basiert wahrscheinlich darauf, daß einerseits die sich im Katheter befindenden elektrolytisch korrodierbaren Stellen des Occlusionsmittels durch den Katheter vom ionischen Medium isoliert sind, also keiner Elektrolyse unterliegen können und andererseits die Stromdichte aufgrund des nach distal zunehmenden Widerstandes in dem Occlusionsmittel von proximal nach distal abnimmt. Die sich nach distal als erstes an das distale Katheterende anschließende elektrolytisch korrodierbar ausgebildete Stelle ist deshalb am stärksten elektrolytischen Prozessen unterworfen und löst sich bevorzugt auf.

Diese Ausführungsform der erfindungsgemäßen Vorrichtung zur Implantation von Occlusionsmitteln kombiniert wie keine bekannte Vorrichtung die Vorteile der Occlusionseffektivität mit operativer Sicherheit und Kostengünstigkeit. Die während des Implantationsvorganges bestimmbare Implantatlänge verhindert, daß für den zu occludierenden Hohlraum ein zu kurzes Occlusionsmittel eingebracht wird, was zur Bildung eines für den zu occludierenden Raum ungenügend großen Thrombus führte. Weiterhin wird verhindert; daß ein hinsichtlich des zu occludierenden Hohlraumes zu langes Occlusionsmittel eingeschoben wird, was die Gefahr von Verletzungen oder Rupturen des zu füllenden Hohlraumes oder angrenzender Gefäße minimiert. Es handelt sich zudem bei der elektrolytischen Abtrennung von Occlusionsmitteln um eine erprobte Technik, deren Parameter weitgehend bekannt sind. Letztlich weist die erfindungsgemäße Vorrichtung zur Implantation von Occlusionsmitteln den Vorteil auf, daß für die Massenfertigung günstige Einheitslängen an Occlusionsmitteln verwendet werden können. Dies stellt einen Kostenvorteil gegenüber den bei der herkömmlichen elektrolytischen oder mechanischen Abtrennung verwendeten Occlusionsmitteln vorbestimmter Länge dar, weil dazu unterschiedlich lange Occlusionsmitteln konfektioniert werden müssen, die dann beim Implantationsvorgang in ihrer Gesamtheit durch Abtrennung der Drahtspitze in den zu occludierenden Hohlraum eingebaut werden.

Um eine möglichst schonende und effektive Ausfüllung des zu occludierenden Körperhohlraumes zu gewährleisten, ist eine Ausbildung der erfindungsgemäßen Vorrichtung vorteilhaft, bei der das Occlusionsmittel oder ein Teil desselben unter elastischer Vorspannung steht, so daß es nach Entlassung aus dem Katheter Windungen ausbildet. Diese Ausbildung erlaubt eine dichte und schonende Ausfüllung des zu occludierenden Hohlraumes, ohne daß das Occlusionsmittel durch die Wandung des zu occludierenden Hohlraumes zur Ausbildung solcher Windungen geformt werden muß, was das Risiko einer Wandungs-Ruptur vermindert. Es kommt dabei im Falle der Ausbildung des Occlusionsmittels als Wendel bzw. Mikrowendel durch die elastische Vorspannung zur Ausbildung sekundärer Windungen.

Um das Occlusionsmittel oder ein Teil desselben mit einer elastischen Vorspannung zu versehen, ist es insbesondere bevorzugt, die Occlusionswendel mit einem Drahtkern aus einem Material mit Formgedächtniseigenschaften zu versehen. Materialien mit Formgedächtniseigenschaften sind beispielsweise Titan und Nickel enthaltene Legierungen, die unter der Bezeichnung Nitinol bekanntgeworden sind, Eisenbasis oder Kupferbasislegierungen. Formgedächtniseigenschaften können auf einer spannungsinduzierten martensitischen Transformation beruhen, wie auch auf einer temperaturinduzierten martensitischen Transformation oder Kombinationen der beiden. Verfahren zur Induzierung von Formgedächtniseigenschaften sind dem Fachmann bekannt. Drähte mit einer Vorspannung dienen in erster Linie als eine Art "Sicherheitsleine", die geeignet ist, die Einholung bereits gesetzter Spiralen (vor der Ablösung) in den Mikrokatheter zu erleichtern.

Wird der Occlusionswendel ein Drahtkern mit einem Materials aus Formgedächtniseigenschaften eingepflanzt, paßt sich die Wendel der aufgegebenen Form des Drahtkerns an. So kommt es zur Ausbildung einer vorgegebenen räumlichen Struktur, die beispielsweise in einer sekundären Windung bestehen kann. Als besonders vorteilhaft haben sich geometrische Käfigstrukturen erwiesen, die einen innen Hohlraum belassen, dergestalt, daß die der Occlusionswendel aufgeprägte Struktur dazu führt, daß diese sich an die Aneurysmawand anlegt. Beispiele für solche Käfigstrukturen sind beispielsweise kugelförmige, Kubus-, Tetraeder- oder Prismastrukturen. Weitere Möglichkeiten und Beispiele sind vorgegebene Strukturen, bei der es zunächst einmal zur Auskleidung der Aneurysmawand mit einer Occlusionswendel kommt und anschließend zu einer Auffüllung des Hohlraums von außen nach innen. Hierzu sind insbesondere sekundäre Spiralstrukturen geeignet, bei denen der Windungsradius größer ist als der Radius des zu behandelnden Aneurysmas.

Eine bevorzugte Form einer Käfigstruktur einer Occlusionswendel beruht auf einer Wendel, deren Verlauf meanderförmig gestaltet ist mit beispielsweise 6 bis 12 und insbesondere 8 bis 10 Bögen. Die Meanderstruktur selbst ist wiederum zu einer räumlichen Struktur verformt, bei der sich die einzelnen Bögen der Occlusionswendel gleichmäßig über die Innenwand einer gedachten Kugel erstrecken, beispielsweise mit einem Durchmesser von 3 bis 20 mm bei einer Gesamtlänge der Spirale von 40 bis 250 mm. Bei Einbringung einer solchen Occlusionswendel in ein beerenförmiges Aneurysma mit ähnlicher oder gleicher Dimension, kommt es zu einer Auskleidung bzw. Verstärkung der Aneurysmawand durch die Spiralstruktur. Der verbleibende Käfig kann mit weiteren Spiralen gefüllt werden.

Die vorstehend beschriebenen Drähte mit superelastischen oder Formgedächtniseigenschaften können durch Kleben, Schweißen, Löten oder mechanisch mit dem Ablöseelement und dem distalen Ende des Occlusionsmittels verbunden sein. Bevorzugt sind Laserschweißen und mechanische Verbindung durch enges Aufwickeln auf das Ablöseelement, analog zur Befestigung der Spiralen/Wendeln auf dem Führungsdraht und Ablöseelement. Occlusionsmittel und Draht bilden auf diese Weise eine konstruktive Einheit, deren Gesamteigenschaften durch die Eigenschaften der Einzelelemente bestimmt werden.

Nach Wegfall des äußeren Zwangs (z. B. durch ein Katheterlumen) kann der Werkstoffverbund aus der Wendel/Spirale (PT oder PT-Legierung) und dem Filament (NiTi) so ausgeführt sein, daß die Auslängkraft von der vorgeformten Struktur zur gelenkten Struktur sowohl maßgebend von den Eigenschaften der Wendel/Spirale wie auch maßgebend von den Eigenschaften des Filaments bestimmt sein kann. So kann beispielsweise der Kraftanteil der Spirale 1 % bis 99 % zur gelenkten Struktur beitragen und der des Filaments 99 % bis 1 %. Vorzugsweise sind die Kraftanteile ausbalanciert, so daß beispielsweise der Anteil der Spirale im Bereich von 25 bis 75 %, insbesondere 40 bis 60 % liegt und der des Filaments bei 75 bis 25 % und insbesondere 60 bis 40 %. Die Variation kann durch die Dimensionierung der Bauteile und/oder durch geeignete Wärmebehandlungen beider Komponenten erfolgen.

Zweckmäßigerweise können der oder die elektrolytisch nicht-korrodierbaren Abschnitte des Occlusionsmittel eines oder mehrere der folgenden Materialien enthalten: Edelmetalle oder Edelmetall-Legierungen, vorzugsweise Platin oder Platin-Legierungen.

Das Ablöseelement/Formteil besteht aus rostfreiem Stahl.

Ferner sind insbesondere die nicht rostenden Stähle der Typen AISI 301, 303 oder 316 bzw. Untergruppen dieser Typen geeignet.

Die für solche Formteile einsetzbaren nicht rostenden Stähle haben vorzugsweise einen Chromanteil X von 12 ≤ x ≤ 20 Gew.-%. Beispiele hierfür sind die Stahlqualitäten 1.4410, 1.4310, 1.4301 und 1.4122. Geeignet sind beispielsweise Chrom/Nickel-Stähle der Qualität 18/8.

Eine weitere Gruppe geeigneter Stähle sind N-legierte austenitische Stähle in nicht rostender Qualität, insbesondere aus der Gruppe der sogenannten druckaufgestickten Stähle.

Austenische Edelstähle der obengenannten Qualitäten können durch eine geeignete Nachbehandlung in ihrem Korrosionsverhalten beeinflußt werden. Insbesondere durch eine Wärmebehandlung können sie in ihrem Gefüge so verändert werden, daß sie für die erfindungsgemäßen Zwecke außerordentlich gut elektrokorrodierbar werden, d. h. durch Anlegen einer elektrischen Spannung in einem Elektrolyten, wie Blut, sich rasch zersetzen. Eine solche Zersetzung findet bei den hier zum Einsatz kommenden Stärken in einer Zeit von weniger als 1 Minute, insbesondere auch in einer Zeit von weniger als 30 Sekunden statt.

Die dazu erforderliche Wärmebehandlung kann mit Hilfe eines Lasers vorgenommen werden, in einem üblichen Ofen oder mittels einer Induktionsspule. Der dazu erforderliche Temperaturbereich liegt bei 500 bis 1000° C, vorzugsweise bei 600 bis 950° C und insbesondere bei 700 bis 900° C. Es wird angenommen, daß bei dieser Wärmebehandlung eine Rekristallisation unter Ausbildung von großen Gefügekörnern und Hartmetallkarbiden stattfindet und damit eine Verringerung Korngrenzenstabilität. Die Ausbildung von Chromkarbiden, die sich auf den Korngrenzen abscheiden, führt zu einer Verarmung der Matrix an Chrom sowie zu einer Herabsetzung des Widerstands gegen interkristalline Korrosion. Die für die Korrosion zur Verfügung stehende Oberfläche wird dadurch rasch erweitert, so daß es zu einer schnellen Zersetzung des Gefüges unter Stromeinfluß in einem Elektrolyt kommt.

Bevorzugt ist eine homogene Ofenwärmebehandlung in den obengenannten Temperaturbereichen, wobei bei höheren Temperaturen die Zeitdauer der Behandlung verkürzt werden kann, aber auch eine Laserwärmebehandlung.

In einer vorteilhaften Ausführungsform werden für die Ausbildung des oder der elektrolytisch nicht-korrodierbaren Abschnitte an den Übergängen zu dem elektrolytisch korrodierbaren Formteil Materialkombinationen gewählt, die dazu geeignet sind, Lokalelemente auszubilden. Auf diese Weise wird - unabhängig von der Verringerung des Durchmessers der korrodierbaren Stellen - die elektrolytische Ablösbarkeit des Occlusionsmittels verbessert.

Hierbei eignen sich am besten Materialkombinationen, in denen zur Ausbildung der elektrolytisch korrodierbaren Stellen nicht rostende Stähle mit Edelmetallen bzw. Edelmetall-Legierungen; insbesondere Pt, Pt-Metalle, Pt-Legierungen, Au-Legierungen oder Sn-Legierungen. Vorzugsweise ist das Formteil beidseitig von Platinwendeln bzw. Wendeln aus Platinlegierungen eingerahmt.

Der Einsatz der vorgenannten Materialien zur Ausbildung des oder der elektrolytisch nicht-korrodierbaren Abschnitte und der elektrolytisch korrodierbaren Stelle oder Stellen des Occlusionsmittels gewährleisten die spezifische elektrolytische Korrosion des Occlusionsmittels an dem dafür bestimmten Formteil in sehr kurzer Zeit.

Die Ausbildung von elektrolytisch korrodierbaren Stellen mit Formteilen, welche zu beiden Seiten Lokalelemente ausbilden, ist besonders vorteilhaft. Diese Ausführungsform der elektrolytisch korrodierbaren Stellen ist wesentlich korrosionsfreudiger und korrodiert daher wesentlich schneller als Formteile, welche nur zu einer Seite ein Lokalelement ausbilden. Bevorzugt sind deshalb Materialkombinationen mit möglichst großem Abstand in der elektrochemischen Spannungsreihe. Auch dies ist ein Vorteil der erfindungsgemäßen Vorrichtung gegenüber den im Stand der Technik vorkommenden Vorrichtungen die zur Ablösung des Embolisierungsdrahtes die Spitze des Führungsdrahtes korrodieren, da in diesem Fall nur zu einer Seite, nämlich zur Seite des Embolisierungsdrahtes (in der Regel ein Platindraht) ein Lokalelement ausgebildet wird.

Die elektrolytisch korrodierbaren Formteile sind zweckmäßigerweise unter funktionellen Gesichtspunkten ausgestaltet. So ist es in Hinsicht auf die Biegebeanspruchung vorteilhaft, die Form des elektrolytisch korrodierbaren Formteils an die Form des Occlusionsmittels anzupassen und dieses beispielsweise in die Windungen eines als Mikrowendel ausgebildeten Occlusionsmittels zu integrieren. Andererseits hat die im wesentlichen gerade Ausbildung des elektrolytisch korrodierbaren Formteils den Vorteil, daß sie technisch unaufwendig ist. Zum Zwecke einer guten Verschieblichkeit des Occlusionsmittels im Katheter ist dabei die Ausrichtung des im wesentlichen geraden elektrolytisch korrodierbaren Formteils in Längsachse des Occlusionsmittels vorteilhaft.

Erfindungsgemäß wird die elektrolytisch korrodierbare Stelle des Occlusionsmittels durch ein Formteil gebildet, das zwischen die elektrolytisch nicht korrodierbaren Anteile des Occlusionsmittels gefügt wird. Diese Ausführungsform hat den Vorteil, daß dabei eine besonders große Vielzahl verschiedener Werkstoffe für die Ausbildung der elektrolytisch korrodierbaren Stelle und der elektrolytisch nicht-korrodierbaren Abschnitte miteinander kombiniert werden können. Diese Ausführungsform weist weiterhin den Vorteil auf, daß die elektrolytisch korrodierbare Stelle und die elektrolytisch nicht korrodierbaren Abschnitte modular und daher technisch unaufwendig aneinandergefügt werden können. Dies ist besonders einfach, wenn die elektrolytisch korrodierbaren Stelle und somit das sie bildende Formteil im wesentlichen gerade ausgebildet ist

Zweckmäßigerweise kann das die elektrolytisch korrodierbare Stelle bildende Formteil mit den nicht-korrodierbaren Abschnitten durch Löt- und/oder Klebe-und/oder Schweißvorgänge verbunden sein. Ebenso kann das die elektrolytisch korrodierbare Stelle bildende Formteil mechanisch an die elektrolytisch nicht korrodierbaren Abschnitte angefügt werden, z. B. durch Einspannung oder Einklemmen, sofern die elektrolytisch nicht korrodierbaren Abschnitte Ausnehmungen zur Aufnahme der Formteile aufweisen. Dies ist z. B. der Fall bei elektrolytisch nicht korrodierbaren Abschnitten, die als einen inneren Hohlraum umschreibende Mikrowendeln ausgebildet sind. Die Formteile können so formschlüssig in diesen Hohlraum eingefügt und fixiert werden. Dabei kann es weiterhin zweckmäßig sein, wenn die die Formteile aufnehmenden Außenbereiche der als Mikrowendel ausgebildeten elektrolytisch nicht korrodierbaren Abschnitte verstärkt sind. Besonders bevorzugt ist es, das Formteil beidseitig mit dem Occlusionsmittel zu verschweißen.

Es ist dabei besonders vorteilhaft, wenn das die elektrolytisch korrodierbare Stelle bildende Formteil durch Ätzen oder andere Verfahren vorkorrodiert ist, so daß sich sein Durchmesser zur Mitte hin verjüngt. Die äußeren bzw. geschwächten Anteile der Formteile mit größerem Durchmesser werden dann durch beispielsweise artfremdes Verschweißen, mechanische Einbringung oder Verklebung an die elektrolytisch nicht korrodierbaren Abschnitte gefügt. Die Verbindung zwischen elektrolytisch korrodierbarer Stelle und elektrolytisch nicht korrodierbaren Abschnitten ist daher sehr stabil, wohingegen der sich infolge der Schwächung zur Mitte des Formteils verjüngende Durchmesser zur guten elektrolytischen Ablösbarkeit der Occlusionsmittel führt. Dabei ist für die Materialkombination von Platin-Legierungen oder Platin-Metallen als Werkstoff für die Ausbildung der elektrolytisch nicht korrodierbaren Abschnitte mit nichtrostendem Stahl als Werkstoff für das die elektrolytisch korrodierbare Stelle bildende Formteil die Verbindung durch artfremdes Verschweißen besonders bevorzugt.

Es kann zweckmäßig sein, das Formteil mit einer teilweisen Beschichtung eines Werkstoffs zu versehen, der in der Spannungsreihe über dem Material steht, welches das Formteil ausbildet. Diese Ausführungsform ist besonders vorteilhaft hinsichtlich elektrolytischen Korrodierbarkeit der elektrolytisch korrodierbaren Stellen, welche dort angesiedelt sind, wo die Beschichtung am Formteil fehlt. Als besonders vorteilhaft haben sich hierbei Beschichtungen von Zn oder Sn bzw. Legierungen dieser Metalle auf Formteilen aus nicht rostendem Stahl herausgestellt.

Die mechanische Anbringung des Formteils ist besonders vorteilhaft, wenn die elektrolytisch korrodierbare Stelle im wesentlichen gerade ausgebildet und entlang der Längsachse der Occlusionsmittel angeordnet ist. Die Aneinanderfügung der die Occlusionsmittel bildenden Module (nicht elektrolytisch korrodierbare Abschnitte und elektrolytisch korrodierbare Stelle) ist in diesem Fall mit einem besonders niedrigen technischen Aufwand verbunden.

Zur weiteren Sicherung und Stabilisierung der Aneinanderfügung der einzelnen, die Occlusionsmittel bildenden Module, kann auch eine Kombination der genannten Verfahren eingesetzt werden.

Die Flexibilität der Occlusionsmittel ist auch bei mechanischer Aneinanderfügung aufgrund der Materialauswahl und aufgrund des geringen Durchmessers der die elektrolytisch korrodierbaren Stellen bildenden Formteile gewährleistet.

In einer Ausführung der erfindungsgemäßen Vorrichtung ist das die elektrolytisch korrodierbare Stellen bildende Formteil als Mikrosystembauteil ausgebildet. Dieses kann beispielsweise als längliche Mikrosystembauteil ausgebildet sein, dessen Durchmesser sich zur Mitte hin verjüngt. Das Einfügen der Mikrosystembauteile erfolgt durch die genannten gängigen Verfahren. Die Verwendung solcher sich zur Mitte hin verjüngender Mikrosystemenbauteile weist dabei den Vorteil auf, daß die Bereiche des größten Durchmessers an die elektrolytisch nicht korrodierbaren Abschnitte gefügt werden und somit einen stabilen Zusammenhalt gewährleisten. Der sich verjüngende Bereich mit geringerem Durchmesser ist dagegen dem umgebenden Medium ausgesetzt und kann leicht elektrolytisch korrodiert werden. Auf diese Art und Weise können besonders geringe Durchmesser der elektrolytisch korrodierbaren Stellen erzielt werden.

Bevorzugte Ausführungsformen für das erfindungsgemäß eingesetzte elektrolytisch korrodierbare Formteil sind beispielsweise Elemente, die aus mehreren Edelstahllitzen geringen Durchmessers bestehen, etwa 2 bis 20 ultradünnen Edelstahllitzen, die auch zu einer Seil- oder Kabelstruktur verflochten sein können. Bevorzugte Mikrostrukturen sind strukturierte Drähte, die beispielsweise Löcher, Kerben, Ausnehmungen oder dergleichen aufweisen, wie auch Mikrostrukturbauteile in Form von Edelstahlprofilteilen mit doppel-T-förmigen, kreuzförmigen, dreiflügeligem oder anderem Querschnitt. Zentrales Ziel ist es, die für die Elektrokorrosion zur Verfügung stehende Oberfläche zu vergrößern. Besonders bevorzugt ist naturgemäß die Kombination solcher Strukturen mit der vorstehend erwähnten Schwächung des Gefüges durch thermische Verfahren, chemische Verfahren oder elektrochemische Verfahren.

Die Verjüngung des Durchmessers der elektrolytisch korrodierbaren Stellen zur Mitte hin ist hinsichtlich guter Korrodierbarkeit auch für andere Ausbildungen der elektrolytisch korrodierbaren Stelle zweckmäßig.

Der Durchmesser des Formteils der erfindungsgemäßen Vorrichtung wird dabei zweckmäßigerweise so gewählt, daß er einerseits hinreichende Stabilität gewährleistet und andererseits die elektrolytisch korrodierbare Stelle in situ elektrolytisch gut korrodierbar sind. Vorteilhaft in dieser Hinsicht sind Ausführungsformen mit an der oder den elektrolytisch korrodierbaren Stellen vorliegenden Durchmessern des Occlusionsmittels zwischen 0,01 bis 0,05 mm, vorzugsweise 0,02 bis 0,04 mm und besonders bevorzugt 0,03 mm. Die elektrolytisch nicht korrodierbaren Abschnitte des Occlusionsmittels können dagegen auch größere Durchmesser aufweisen.

In einer weiteren vorteilhaften Ausführungsform ist die Spitze des Führungsdrahtes und/oder des daran angrenzenden Teils des Occlusionsmittels bis hin zum Formteil beispielsweise durch eine schlecht korrodierbare Materialbeschichtung oder einen Überzug mit einem Schrumpfschlauch isoliert, so daß sie nicht elektrolytisch angegriffen wird.

Die erfindungsgemäße Vorrichtung weist zweckmäßigerweise einen röntgendichten Marker auf, der insbesondere distal zum Führungsdraht ausgebildet ist. Vorzugsweise besteht der Marker aus dem proximalen Ende es Occlusionsmittels, das zwischen Führungsdraht und Formteil angeordnet ist und wird von einer Wendel aus Platin oder einem Platinmetall gebildet. Besonders bevorzugt ist eine definierte Länge dieser Wendel von beispielsweise 1 cm oder 3 cm, so daß sie zur punktgenauen Positionierung der Wendel verwandt werden kann. Dazu wird die Markierung mit einer weiteren Markierung im distalen Bereich eines Mikrokatheters in Übereinstimmung gebracht, so daß sichergestellt ist, daß die Ablösestelle in Form des Formteils sich außerhalb des Mikrokatheters befindet und unter dem Einfluß einer angelegten Spannung dem Korrosionsvorgang unterliegt. Die Plazierung des Mikrokatheters selbst in Relation zum Einsatzort des Occlusionsmittels erfolgt auf konventioneller Weise über die Markierung des Mikrokatheters.

Die erfindungsgemäße Vorrichtung ist vorzugsweise für den Einsatz in tier- oder humanmedizinischen Verfahren, besonders bei der endovaskulären Behandlung intrakranieller Aneurysmen und erworbener oder angeborener arteriovenöser Gefäßmißbildungen und/oder -fisteln und/oder Tumorembolisation durch Thrombosierung bestimmt.

Die Erfindung wird nachfolgend beispielhaft anhand der in den Zeichnungen veranschaulichten Ausführungsbeispiele näher erläutert. Es stellen dar:
- Figur 1a: die Vertikalansicht einer in ein Beerenaneurysma positionierten Mikrowendel 3 mit dazugehöriger Vorrichtung in vielfacher Vergrößerung;
- Figur 1b: die Ausschnitts-Vergrößerung 15 aus Figur 1 in dem Bereich der elektrolytisch korrodierbaren Stelle 9 der Mikrowendel 3;
- Figur 2a bis c: in gegenüber Figur 1 stärkerer Vergrößerung drei Möglichkeiten der Anordnung elektrolytisch korrodierbarer Stellen 9 und elektrolytisch nicht korrodierbarer Abschnitte 10 in der Mikrowendel 3;
- Figur 3a bis c: in gegenüber Figur 1 stärkerer Vergrößerung drei Möglichkeiten der Anordnung von elektrolytisch korrodierbarer Stellen 11 und elektrolytisch nicht korrodierbarer Abschnitte 10 in der Mikrowendel 3 mit entlang der Längsachse der Mikrowendel 3 angeordneten elektrolytisch korrodierbarer Stelle 9;
- Figur 4: eine Längsschnittansicht der Anbringung des Führungsdrahtes 2 an die Mikrowendel 3
- Figur 5: eine Darstellung eines Occlusionsmittels mit Occlusionswendel und Ablösestelle 11; und
- Figur 6a und b: eine Occlusionswendel mit einer aufgeprägten Meanderstruktur in flächiger Darstellung (a) und in räumlicher Darstellung (b).

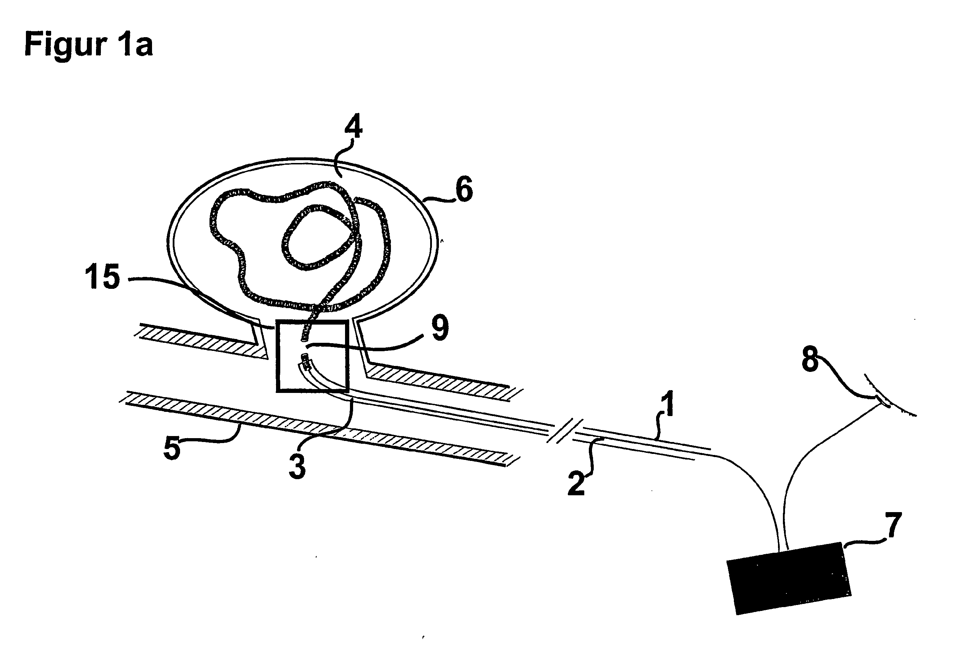

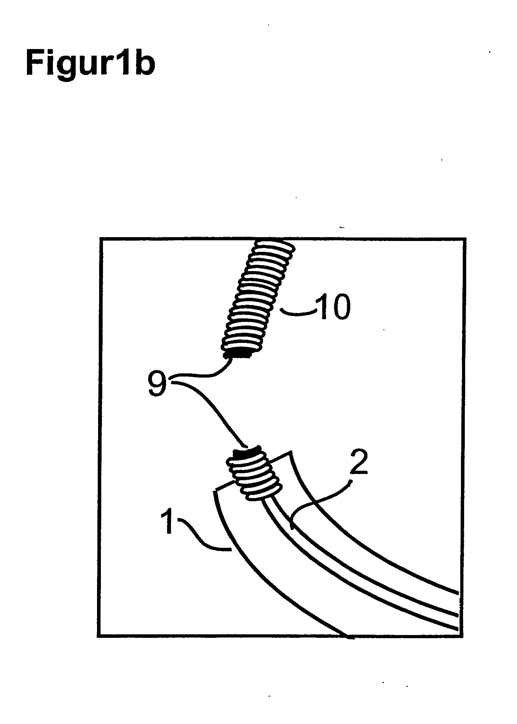

In der Figur 1a ist mit 1 ein Katheter, insbesondere ein biegsam ausgebildeter Mikrokatheter bezeichnet. Durch den Mikrokatheter 1 wird das aus einer Platinmetall-Legierung gefertigte, mit einer elektrolytisch korrodierbaren Stelle 9/11 aus Edelstahl versehene und als Mikrowendel ausgebildetes Occlusionsmittel 3 mit Hilfe des schweißtechnisch an die Mikrowendel 3 gefügten Führungsdrahtes 2 an dem Eingang des Aneurysmas 6 positioniert. Da die durch artfremdes Schweißen gefügte Verbindung zwischen dem Führungsdraht 2 und dem ersten, proximalen, nicht elektrolytisch korrodierbaren Abschnitt der Mikrowendel 3 nicht zur elektrolytischen Ablösung der Mikrowendel 3 bestimmt ist und folglich nicht von besonders geringem Durchmesser sein muß, kann sie besonders stabil ausgebildet sein. Die Verwendung von nichtrostendem Stahl und Platin für Ausbildung des Führungsdrahtes 2 einerseits bzw. der Mikrowendel 3 andererseits ist dabei besonders vorteilhaft, da der in Stahl enthaltene Nickel sich beim Verschweißen zu einer sehr glatten und stabilen Verbindung mit dem Platin fügt. Durch die in Längsachse des Mikrokatheters 1 nach distal erfolgende Verschiebung des Führungsdrahtes 2 erfolgt die Einführung der Mikrowendel 3 in das Aneurysma 6, welche aufgrund ihrer elastischen Vorspannung nach Verlassen des Mikrokatheters 1 sekundäre Windungen 4 ausbildet. Durch die Längsverschieblichkeit von Führungsdraht 2 und Mikrowendel 3 im Mikrokatheter 1 wird der zu implantierende Teil der Mikrowendel 3 in das Aneurysma 6 eingebracht und die elektrolytisch korrodierbare Stelle 9 außerhalb des Mikrokatheters 1 im Blutstrom angeordnet. Die korrekte Positionierung wird dabei unter beispielsweise röntgenologischer Kontrolle gemäß geeigneter Verfahren des Standes der Technik vorgenommen. Anschließend wird mit Hilfe der Spannungsquelle 7, der auf der Körperoberfläche positionierten Kathode 8 und der als Anode dienenden im zu occludierenden Aneurysma 6 positionierten Mikrowendel 3 eine Spannung über einen Zeitraum von 0,1 - 20 Minuten angelegt. Dadurch wird die elektrolytische Abtrennung des sich im Blut befindenden Teils der Mikrowendel 3 an der im Blutstrom sich befindenden elektrolytisch korrodierbaren Stelle 9 vorgenommen. Figur 1 stellt eine Mikrowendel 3 dar, deren dem distalen Ende des Mikrokatheters 1 nächstliegende elektrolytisch korrodierbare Stelle 9 bereits elektrolytisch korrodiert wurde. Mit 15 ist der Bereich gekennzeichnet, welcher in der Figuren 1b vergrößert dargestellt ist.

Die Figur 1b stellt in Ausschnittsvergrößerung der Figur 1 die außerhalb des Mikrokatheters 1 positionierte, bereits elektrolytisch korrodierte elektrolytisch korrodierbare Stelle 9 dar. Das Occlusionsmittel 3 ist hier zunächst über einen elektrolytisch nicht korrodierbaren Abschnitt 10 an den Führungsdraht 2 angebracht. Nach distal schließt sich an die elektrolytisch korrodierbare Stelle 9 des Occlusionsmittels 3 ein weiterer elektrolytisch nicht korrodierbarer Abschnitt 10 an, welcher zusammen mit dem daran verbleibenden Rest der elektrolytisch korrodierten elektrolytisch korrodierbaren Stelle 9 im Aneurysma deponiert wird. Die elektrolytisch korrodierbare Stelle 9 ist in diesem Beispiel an die Form der Mikrowindungen 19 der Mikrowendel 3 angepaßt.

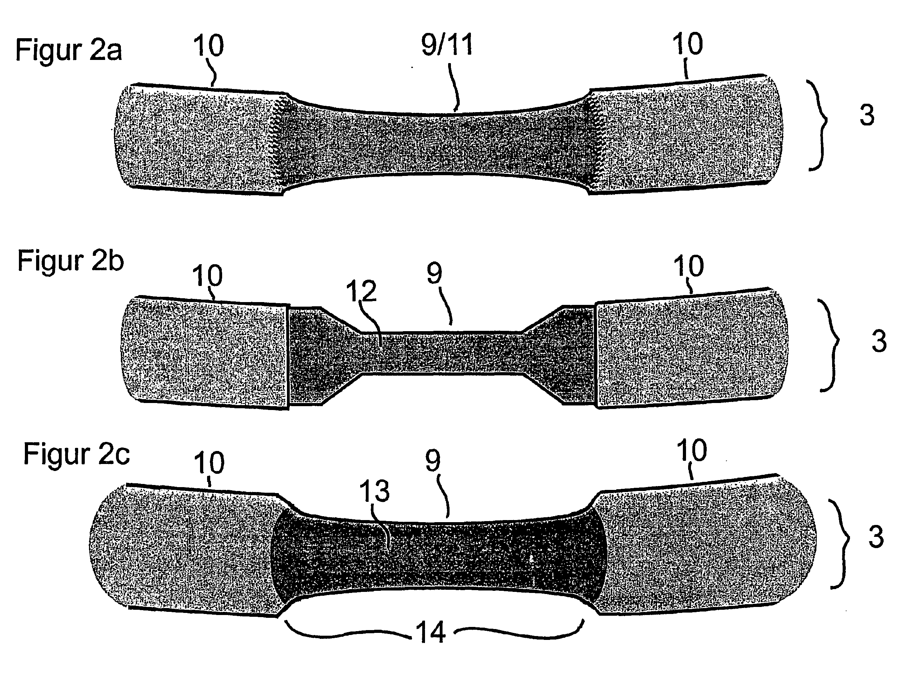

Die Figuren 2a bis c stellen in gegenüber Figur 1 stärkerer Vergrößerung einen Ausschnitt dreier unterschiedlicher Ausführungsformen der erfindungsgemäßen Mikrowendel 3 dar.

Die Figur 2a zeigt eine Mikrowendel 3 mit einem oder mehreren nicht-korrodierbaren Abschnitten 10 aus einer Platinmetall-Legierung, an die schweißtechnisch eine aus Edelstahl bestehende die elektrolytisch korrodierbare Stelle 9 bildendes Formteil 11 von 0,03 mm Durchmesser angefügt ist.

Die Figur 2b zeigt einen Ausschnitt aus einer erfindungsgemäßen Mikrowendel 3 mit einem Mikrosystembaustein 16 als elektrolytisch korrodierbare Stelle 11, welcher durch einen Klebevorgang zwischen die nicht elektrolytisch korrodierbaren Abschnitte 10 eingefügt ist.

Die Figur 2c stellt einen Ausschnitt einer einen Edelstahlkern 13 von 0,03 mm Durchmesser enthaltenden Mikrowendel 3 dar. Dieser Edelstahlkern 12 ist von einer Beschichtung aus elektrolytisch-korrosionsbeständigem Material 14 umgeben, die in regelmäßigen Abständen mit Unterbrechungen versehen ist, an denen der Edelstahlkern 13 von außen zugänglich ist und dementsprechend eine elektrolytisch korrodierbare Stelle 9 ausbildet.

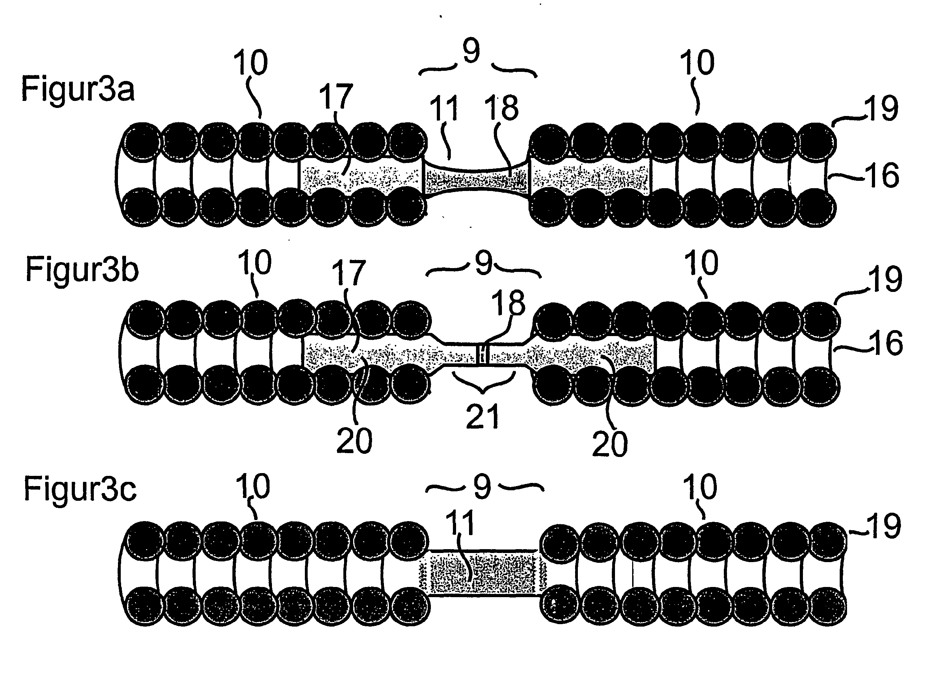

Die Figuren 3a-c stellen in gegenüber Figur 1 stärkerer Vergrößerung einen Ausschnitt dreier unterschiedlicher Ausführungsformen der erfindungsgemäßen Mikrowendel 3 mit in der Längsachse der Mikrowendel 3 ausgerichteten elektrolytisch korrodierbarer Stelle oder Stellen 9 dar.

Die Figur 3a zeigt eine Mikrowendel 3, mit einem im wesentlichen geraden Formteil11 aus nicht rostendem Stahl, welches formschlüssig in den Innenraum 16 der Mikrowindungen 19 aus Platindraht eingefügt ist. Die modulare Aneinanderfügung von elektrolytisch nicht korrodierbaren Abschnitten 10 aus Platindraht-Mikrowindungen 19 und im wesentlichen geraden Formteilen 11 führt dort, wo das im wesentlichen gerade Formteil 11 nicht von den Platinmetall-Mikrowindungen 19 umgeben und somit von außen zugänglich ist, zur Ausbildung einer elektrolytisch korrodierbaren Stelle 9. Die nicht korrodierbaren Abschnitte 10 der Mikrowendel 3 werden dagegen durch den in Mikrowindungen 19 gewundenen Platindraht gebildet, welcher sich jeweils mechanisch formschlüssig zu beiden Seiten um das im wesentlichen gerade ausgebildete Edelstahlformteil 11 schließt. Das Formteil 11 ist von einer Sn-Schicht 17 umgeben, welche in der Mitte infolge der Vorkorrodierung abgelöst ist. Infolge dessen ist der die elektrolytisch korrodierbare Stelle 9 ausbildende vorkorrodierte Mittelteil 18 des Formteils 11 der elektrolytischen Korrosion besonders gut zugänglich, da er einen sehr geringen Durchmesser aufweist und zu beiden Seiten infolge der Sn-Beschichtung 17 Lokalelemente ausbildet.

Die Figur 3b stellt ebenfalls eine modular aufgebaute Mikrowendel 3 dar, in der sich die Mikrowindungen 19 des Platindrahtes formschlüssig um die Enden eines aus Edelstahl gefertigten Mikrosystembausteins 11 schließen und so die elektrolytisch nicht korrodierbaren Abschnitte 10 bilden, zwischen denen der freiliegende Abschnitt des Mikrosystembausteins 11 die elektrolytisch korrodierbare Stelle 9 ausbilden. Die Einfügung der Abschnitte des Mikrosystembausteins 11 mit größerem Durchmesser 20 in den Innenraum 16 der Platindrahtmikrowindungen 19 gewährleistet eine stabile Fixierung der modularen Elemente aneinander. Die Ausbildung der elektrolytisch korrodierbaren Stelle 9 durch den verjüngten Abschnitt 21 des Mikrosystembausteins 11 mit geringerem Durchmesser ermöglicht dagegen ein großes Maß an Flexibilität sowie vorteilhaft gute Korrodierbarkeit der elektrolytisch korrodierbaren Stelle 9.

Diese Korrodierbarkeit wird verstärkt durch den Aufbau des Mikrosystembausteins, welcher zum großen Teil aus einer Sn-Legierung 17 besteht und lediglich in der sich verjüngenden Mitte ein Mikroablöseelement 22 enthält, welches aus nicht rostendem Stahl besteht und eine extrem kleine elektrolytisch korrodierbare Stelle 9 ausbildet. Diese vorteilhafte Ausbildungsform ist extrem leicht korrodierbar und daher besonders gut ablösbar.

In Figur 3c ist alternativ der Ausschnitt aus einer erfindungsgemäßen Mikrowendel 3 mit einem im wesentlichen geraden die elektrolytisch korrodierbare Stelle 9 bildenden Formteil 11 aus Edelstahl gezeigt, das schweißtechnisch an die die nicht korrodierbaren Abschnitte 10 bildenden Mikrowindungen 19 aus Platindraht gefügt ist.

Die Elektronegativitäts-Unterschiede zwischen der die korrodierbare Stelle 9 ausbildenden Edelstahl und der die nicht-korrodierbaren Abschnitte 10 ausbildenden Platinmetall-Legierung bewirkt in einem ionischen Medium wie dem Blut bei Anlegen einer elektrischen Spannung die elektrolytische Auflösung der elektrolytisch korrodierbaren Stelle 9.

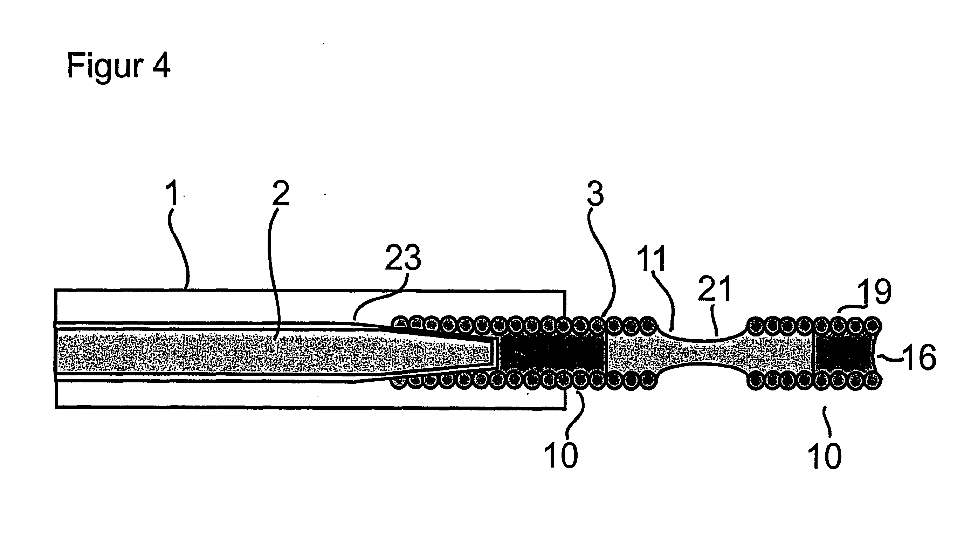

Die Figur 4 stellt einen Längsschnitt durch den Übergang zwischen dem Führungsdraht 2 und der Occlusionswendel 3 in Vergrößerung dar. In diesem Beispiel ist der Führungsdraht 2 aus stabilem nicht rostendem Stahl ausgebildet und von einer Beschichtung 23 umgeben, welche die Korrosion des Stahls verhindert. Diese Beschichtung kann entweder nicht leitend ausgebildet sein, in diesem Falle wird der elektrische Strom für das Ende des Mikrokatheters 1 an die Occlusionswendel 3 geleitet. Oder die Beschichtung ist nicht korrodierbar aber elektrisch leitend (beispielsweise eine Graphitbeschichtung) in welchem Fall der elektrische Strom auch über den Führungsdraht 2 in die Mikrowendel 3 geleitet werden kann. In diesem Beispiel wird die Mikrowendel 3 durch eine Anordnung eines vorkorrodierten Formteils 11 aus nicht rostendem Stahl zwischen elektrolytisch nicht korrodierbare Abschnitte 10 aus Platindraht gebildet. Das Formteil 11 ist mit seinen Enden mechanisch in den Innenraum 16 der Platindraht Mikrowindungen 19 gefügt. Das in der Mitte vorkorrodierte Formteil 11 bildet aufgrund des geringen Durchmessers 21 eine elektrolytisch besonders gut korrodierbare Stelle aus.

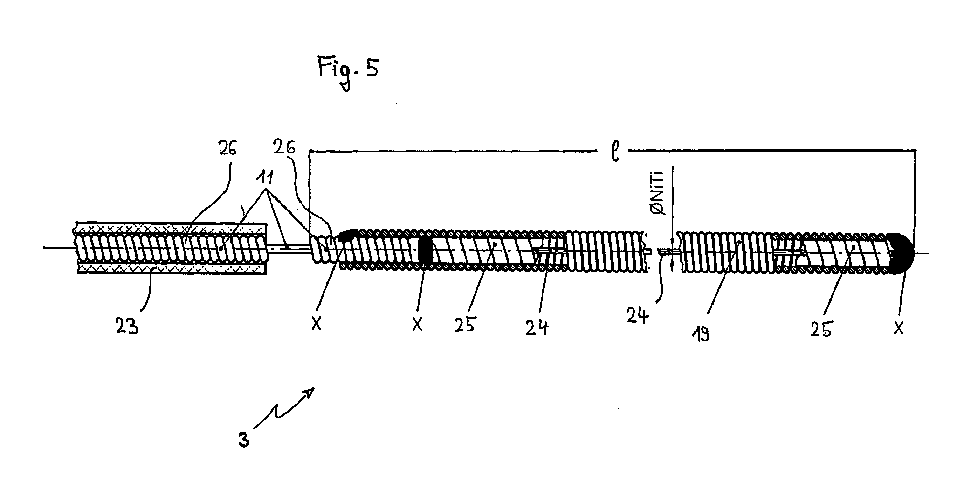

Figur 5 zeigt eine Occlusionswendel 3 mit dem integrierten Ablöseelement 11, das beidseitig von Verstärkungsspiralen 26 eingefaßt wird. Proximal ist die Verstärkungsspirale 26 mit einer Beschichtung 23 versehen, distal schließt sich an die Verstärkungsspirale 26 die eigentliche Occlusionsspirale 19 an.

Nach der Abtrennung der Occlusionsspirale an der Trennstelle durch Elektrokorrosion des Trennelements 11 verbleibt die Länge L des Implantats am Implantationsort. Die Occlusionswendel selbst weist neben der eigentlichen Wendel aus Platin oder einer Platinlegierung 19 einen zentralen Draht 24 auf, in diesem Fall aus Nickel-Titan-Legierung mit superelastischen oder Formgedächtniseigenschaften, der als innere Sicherheitsleine dient bzw. der Spirale eine vorgegebene räumliche Struktur aufgeben kann. Der Draht 24 ist über Laserschweißstellen X am proximalen und distalen Ende der Occlusionswendel festgelegt. Endständige Spiralhülsen 25 dienen der Verstärkung der Occlusionswendel in den Endbereichen. Die Occlusionswendel 19 ist zusätzlich durch eine Laserschweißstelle X an der Verstärkungsspirale 26 gesichert. Die Verstärkungsspiralen 26 sind vorzugsweise aus Edelstahl gefertigt.

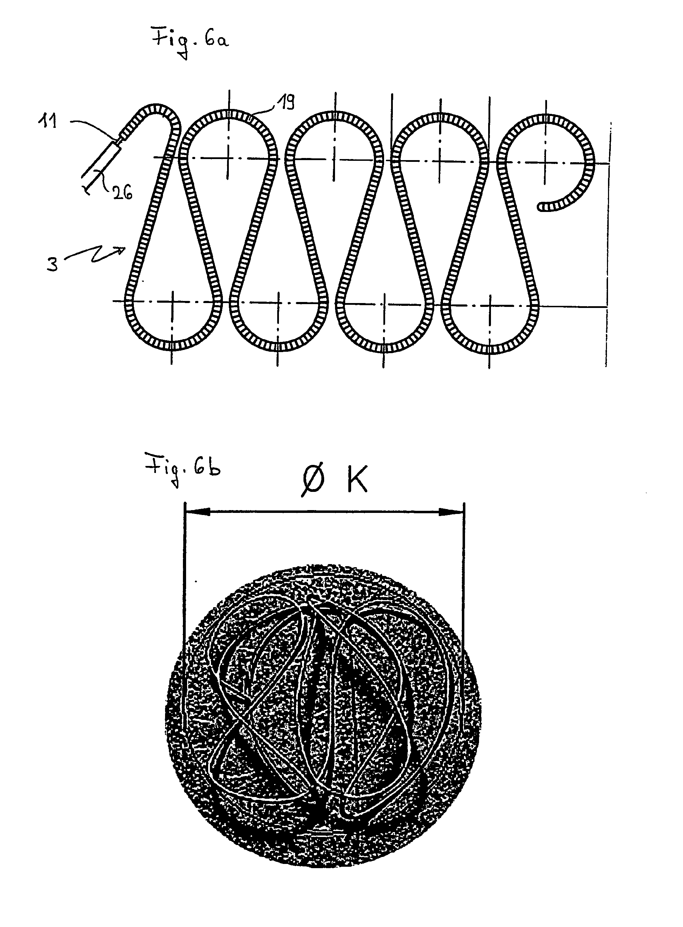

Figur 6a zeigt eine Occlusionswendel 3 mit der Spirale 19, der Ablösestelle 11 und die mit einer Beschichtung versehenen proximalen Verstärkungswendel 26. Die Occlusionsspirale selbst weist, auf die Fläche bezogen, eine meanderförmige Struktur mit insgesamt 8 Bögen auf. Die Struktur ist durch einen Formgedächtniseffekt des in der Spirale enthaltenen Nitinoldrahtes vorgegeben und wird eingenommen, sobald die Spirale vom Zwang des umgebenden Mikrokatheters befreit ist. Bei einer Gesamtlänge von etwa 240 mm haben die Bögen beispielsweise einen Durchmesser von 10 mm. Der Durchmesser der Spirale kann beispielsweise bei 0,15 mm liegen, der des Nitinoldrahtes bei 0,05 mm.

Die Occlusionswendel hat vorzugsweise an ihrem distalen und proximalen Ende einen gekrümmten Verlauf, um einen Stiletteffekt zu vermeiden. Zusätzlich ist das distale Ende, wie in Figur 5 gezeigt, mit einem Schweißpunkt rund ausgebildet.

Figur 6b zeigt die der Spirale von Figur 6a aufgegebene eigentliche räumliche Struktur, die diese einnimmt, wenn sie in einem Aneurysma freigesetzt wird. In ihrer räumlichen Struktur lehnt sich die Spirale an die Innenwande einer Kugel an und bildet dort die aufgeprägten Schlaufen aus. Der Durchmesser der Kugel beträgt im gezeigten Fall etwa 20 mm.

Die in Figur 6b dargestellte räumliche Struktur kann der gezeigten Occlusionswendel mit Hilfe eines Werkzeugs aufgeprägt werden, das die Form eines Zylinders oder einer Kugel hat. Dabei wird die Occlusionswendel in Meanderform über die Oberfläche des Werkzeuges gelegt, dort fixiert und einem üblichen Formgebungsverfahren in einem Ofen unterworfen. Der Materialverbund aus zentralem Nitinoldraht und peripherer Wendel behält nach Brennen die ihr vorgegebene Form bei und trachtet danach, diese erneut einzunehmen, wenn sie aus einem Mikrokatheter freigesetzt wird.

## Patentansprüche

1. Vorrichtung zur Implantation von durch Elektrolyse ablösbaren Okklusionswendeln in Körperhohlräumen oder Blutgefäßen mit einem Führungsdraht (2) und einem distal daran angeordneten Okklusionsmittel (3), wobei das Okklusionsmittel (3) eine Okklusionswendel (3) mit wenigstens einer elektrolytisch korrodierbaren Stelle aufweist, und die elektrolytisch korrodierbaren Stellen Formteile (9) aus nicht rostendem Stahl oder anderen korrodierbaren Materialien sind, **dadurch gekennzeichnet, dass** die Formteile (9) durch eine Wärmebehandlung bei einer Temperatur im Bereich von 500 bis 1.000°C vorkorrodiert wurden.

2. Vorrichtung nach Anspruch 1, **dadurch gekennzeichnet, dass** die elektrolytisch nicht korrodierbaren Abschnitte (10) der Okklusionswendel aus Edelmetall oder Edelmetall-Legierungen bestehen, insbesondere aus Platin oder Platinmetall-Legierungen.

3. Vorrichtung nach Anspruch 1 oder 2, **dadurch gekennzeichnet, dass** das Formteil aus nicht rostendem Stahl mit einem Chromanteil x von 12 ≤ = x ≤ = 20 Gew.-% besteht.

4. Vorrichtung nach Anspruch 3, **dadurch gekennzeichnet, dass** der nicht rostende Stahl ein Stahl der Qualität 1.4410, 1.4310, 1.4301 oder 1.4122 ist.

5. Vorrichtung nach Anspruch 1 oder 2, **dadurch gekennzeichnet, dass** nicht rostender Stahl der Typen AISI 301, 304 oder 316 oder Untergruppen derselben zur Ausbildung des elektrolytisch korrodierbaren Formteils (9) verwendet wird.

6. Vorrichtung nach Anspruch 1 oder 2, **dadurch gekennzeichnet, dass** das Formteil aus einem N-legierten austenitischen Stahl, insbesondere druckaufgestickten austenitischen Stahl besteht.

7. Vorrichtung nach einem der vorstehenden Ansprüche, **dadurch gekennzeichnet, dass** die Vorkorrosion durch eine Wärmebehandlung in einem Ofen erfolgt ist.

8. Vorrichtung nach einem der vorstehenden Ansprüche, **dadurch gekennzeichnet, dass** das Formteil (9, 11) von Elementen umgeben ist, die in der Spannungsreihe über dem Material des Formteils stehen, so dass sich Lokalelemente ausbilden.

9. Vorrichtung nach Anspruch 8, **dadurch gekennzeichnet, dass** die Elemente aus Edelmetall oder Edelmetall-Legierungen, insbesondere aus Platin oder Platinmetall-Legierungen bestehen.

10. Vorrichtung nach Anspruch 8 oder 9, **dadurch gekennzeichnet, dass** die Elemente Mikrowendeln aus Platin oder Platinmetall-Legierungen sind.

11. Vorrichtung nach einem der vorhergehenden Ansprüche, **dadurch gekennzeichnet, dass** die Form der elektrolytisch korrodierbaren Formteile (9) an die Mikrowindungen (19) der Okklusionswendeln (3) angepasst sind.

12. Vorrichtung nach einem der vorhergehenden Ansprüche, **dadurch gekennzeichnet, dass** das elektrolytisch korrodierbare Formteil (11) mit der Okklusionswendel (3) über Schweißverbindungen, vorzugsweise durch Laserschweißen, verbunden ist.

13. Vorrichtung nach einem der vorhergehenden Ansprüche, **dadurch gekennzeichnet, dass** das distale Ende des Führungsdrahtes (2) und/oder der proximal zum Formteil (9) angeordneten Teil des Okklusionsmittel (3) mit einer die Korrosion verhindernden Beschichtung (23) versehen ist.

14. Vorrichtung nach Anspruch 1, **dadurch gekennzeichnet, dass** das Okklusionsmittel (3) einen zentralen Draht aufweist, der das Formteil mit dem distalen Teil des Okklusionsmittels (3) verbindet und aus superelastisch eingestelltem Material besteht.

15. Vorrichtung nach einem der vorstehenden Ansprüche, **dadurch gekennzeichnet**, da22 sie weiterhin einen Mikrokatheter aufweist.

16. Vorrichtung nach Anspruch 15, **dadurch gekennzeichnet, dass** der Mikrokatheter wenigstens ein Markerelement in seinem distalen Bereich aufweist.

17. Vorrichtung nach einem der vorsehenden Ansprüche, **dadurch gekennzeichnet, dass** sie einen Katheter (1), eine Spannungsquelle (7) und eine Kathode (8) aufweist, wobei die Okklusionswendel (3) in dem Katheter (1) in Längsrichtung verschiebbar ist und als Anode dient, so dass im Kontakt mit einer Körperflüssigkeit der distal zum Formteil (9) befindliche Teil der Okklusionswendel (3) durch Elektrolyse abgetrennt werden kann.

## Claims

1. Device for the implantation of occlusion means severable by electrolysis in body cavities or blood vessels comprising a guide wire (2) and an occlusion means (3) arranged at its distal end, wherein the occlusion means (3) comprises an occlusion coil (3) with at least one electrolytically corrodible section, the electrolytically corrodible sections being form pieces (9) of stainless steel or other corrodible materials, **characterized in that** the form pieces (9) are precorroded by heat treatment at a temperature in the range of 500°C to 1000°C.

2. Device according to claim 1, **characterized in that** the electrolytically non-corrodible sections (10) of the occlusion coil consist of noble metal or noble metal alloys, in particular platinum or platinum metal alloys.

3. Device according to claim 1 or 2, **characterized in that** the form piece consists of stainless steel having a chromium content x of 12 ≤ = x ≤ = 20 % by weight.

4. Device according to claim 3, **characterized in that** the stainless steel is of material grade 1.4410, 1.4310, 1.4301 or 1.4122.

5. Device according to claim 1 or 2 , **characterized in that** stainless steel of type AISI 301, 304 or 316 or subgroups thereof is employed to produce the electrolytically corrodible form piece (9).

6. Device according to claim 1 or 2, **characterized in that** the form piece consists of an N-alloyed austenitic steel, particularly of austenitic steel nitrogen-hardened under pressure.

7. Device according to any one of the preceding claims, **characterized in that** pre-corrosion has been effected by a heat treatment carried out in a furnace/oven.

8. Device according to any one of the preceding claims, **characterized in that** form piece (9, 11) is surrounded by elements ranking higher in the electrochemical series than the material of which the form piece is made so that local elements are produced.

9. Device according to claim 8, **characterized in that** the elements are of noble metal or noble metal alloys, in particular of platinum or platinum metal alloys.

10. Device according to claim 8 or 9, **characterized in that** the elements are micro-coils made of platinum or platinum metal alloys.

11. Device according to any one of the preceding claims, **characterized in that** the shape of the electrolytically corrodible form pieces (9) is adapted to the micro-turns (19) of the occlusion coils (3).

12. Device according to any one of the preceding claims, **characterized in that** the electrolytically corrodible form piece (11) is connected to the occlusion coil (3) by means of welding joints, preferably by a laser welding method.

13. Device according to any one of the preceding claims, **characterized in that** the distal end of the guide wire (2) and/or the portion of the occlusion means (3) arranged proximally to the form piece (9) has been provided with a corrosion-preventing coating (23).

14. Device according to claim 1, **characterized in that** the occlusion means (3) comprises a central wire that connects the form piece with the distal portion of the occlusion means (3) and consists of a material having superelastic characteristics.

15. Device according to any one of the preceding claims, **characterized in that** it, furthermore, comprises a micro-catheter.

16. Device according to claim 15, **characterized in that** the micro-catheter has at least one marker element located in its distal area.

17. Device according to any one of the preceding claims, **characterized in that** it comprises a catheter (1), a voltage source (7) and a cathode (8), with the occlusion coil (3) in the catheter (1) being slidable in longitudinal direction and serving as anode so that when in contact with a body fluid the portion of the occlusion coil (3) arranged distally to the form piece (9) can be severed by electrolysis.

## Revendications

1. Dispositif pour implanter des spires d'occlusion détachables par électrolyse dans des cavités corporelles ou des vaisseaux sanguins, avec un fil de guidage (2) et un moyen d'occlusion (3) placé sur celui-ci en position distale, dans lequel le moyen d'occlusion (3) présente une spire d'occlusion (3) ayant au moins un endroit corrodable électrolytiquement et les endroits corrodables électrolytiquement sont des parties moulées (9) en acier inoxydable ou d'autres matériaux corrodables ; les pièces moulées (9) ayant été pré-corrodées par traitement thermique à une température de 500°C à 1000°C.

2. Dispositif selon la revendication 1, **caractérisé en ce que** les sections (10) de la spire d'occlusion, non corrodables électrolytiquement, sont constituées d'un métal noble ou d'alliages de métaux nobles, en particulier de platine ou d'alliage de platine.

3. Dispositif selon la revendication 1 ou 2, **caractérisé en ce que** les pièces moulées sont constituées d'un acier inoxydable ayant une teneur en chrome de 12 ≤ à ≤ 20 % en poids.

4. Dispositif selon la revendication 3, **caractérisé en ce que** l'acier inoxydable est un acier de qualité 1.4410, 1.4310, 1.4301 ou 1.4122.

5. Dispositif selon la revendication 1 ou 2, **caractérisé en ce que** de l'acier inoxydable de type AISI 301, 304 ou 316 ou leurs sous-groupes est utilisé pour former la pièce moulée corrodable électrolytiquement (9).

6. Dispositif selon la revendication 1 ou 2, **caractérisé en ce que** la pièce moulée est constituée d'un acier austénitique allié avec N, en particulier d'acier austénitique azoté sous pression.

7. Dispositif selon l'une des revendications précédentes, **caractérisé en ce que** la pré-corrosion est obtenue par un traitement thermique dans un four.

8. Dispositif selon l'une des revendications précédentes, **caractérisé en ce que** la pièce moulée (9, 11) est entourée d'éléments qui se trouvent au-dessus du matériau de la pièce moulée dans la série des contraintes, de sorte qu'il se forme des éléments locaux.

9. Dispositif selon la revendication 8, **caractérisé en ce que** les éléments sont constitués d'un métal noble ou d'alliages de métaux nobles, en particulier de platine ou d'alliages de platine.

10. Dispositif selon la revendication 8 ou 9, **caractérisé en ce que** les éléments sont des micro-spires en platine ou alliage de platine.

11. Dispositif selon l'une des revendications précédentes, **caractérisé en ce que** la forme des pièces moulées corrodables électrolytiquement (9) est adaptée aux micro-enroulements (19) des spires d'occlusion (3).

12. Dispositif selon l'une des revendications précédentes, **caractérisé en ce que** la pièce moulée corrodable électrolytiquement (11) est reliée à la spire d'occlusion (3) par l'intermédiaire de soudures, de préférence par soudage laser.

13. Dispositif selon l'une des revendications précédentes, **caractérisé en ce que** l'extrémité distale du fil de guidage (2) et/ou de la partie du moyen d'occlusion (3) située en position proximale par rapport à la pièce moulée (9) est munie d'un revêtement (23) empêchant la corrosion.

14. Dispositif selon la revendication 1, **caractérisé en ce que** le moyen d'occlusion (3) présente un fil central qui relie la pièce moulée à la partie distale du moyen d'occlusion (3) et est constitué d'un matériau réglé super-élastique.

15. Dispositif selon l'une des revendications précédentes, **caractérisé en ce qu'**il présente de plus un micro-cathéter.

16. Dispositif selon la revendication 15, **caractérisé en ce que** le micro-cathéter présente au moins un élément marqueur dans sa partie distale.

17. Dispositif selon l'une des revendications précédentes, **caractérisé en ce qu'**il présente un cathéter (1), une source de tension (7) et une cathode (8) ; la spire d'occlusion (3) étant mobile longitudinalement dans le cathéter (1) et servant d'anode, de sorte qu'au contact d'un liquide corporel, la partie de la spire d'occlusion (3) située en position distale par rapport à la pièce moulée (9) peut être séparée par électrolyse.
